# EUROPÄISCHE PATENTSCHRIFT

(11) **EP 1 268 417 B1**
(45) Veröffentlichungstag und Bekanntmachung des Hinweises auf die Patenterteilung: **21.03.2007**
(21) Anmeldenummer: 01915404.6
(22) Anmeldetag: 22.03.2001
(51) Int. Cl.: C07C 409/10, C07C 409/08, C07C 409/14, C07C 45/53, C07C 37/08

(54) **VERFAHREN ZUR HERSTELLUNG VON AROMATISCHEN ALKOHOLEN, INSBESONDERE PHENOL**
METHOD FOR PRODUCING AROMATIC ALCOHOLS, ESPECIALLY PHENOL
PROCEDE POUR LA PRODUCTION D'ALCOOLS AROMATIQUES, NOTAMMENT DE PHENOL

(30) Priorität: 30.03.2000 DE 10015874
(43) Veröffentlichungstag der Anmeldung: 02.01.2003
(73) Patentinhaber: Degussa GmbH, 40474 Düsseldorf (DE)
(72) Erfinder: KÜHNLE, Adolf, 45770 Marl (DE); DUDA, Mark, 67071 Ludwigshafen (DE); TANGER, Uwe, 44879 Bochum (DE); SHELDON, Roger Arthur, NL-2281 VA Rijswijk (NL); ARENDS, Isabella, W., C., E., NL-2548 SL's Gravenhage (NL); SASIDHARAN, Manickam, Tamil Nadu 606 604 (IN)
(86) Internationale Anmeldenummer: PCT/EP2001/003288
(87) Internationale Veröffentlichungsnummer: WO 2001/074767

(56) Entgegenhaltungen:
- EP-A- 0 198 351
- EP-A- 0 927 717

## Beschreibung

Die Erfindung betrifft ein Verfahren zur Herstellung von aromatischen Alkoholen, insbesondere Phenol durch katalytische Oxidation von aromatischen Kohlenwasserstoffen zu den entsprechenden Hydroperoxiden und anschließende Spaltung der Hydroperoxide.

Um zu Hydroxylgruppen enthaltenden aromatischen Verbindungen wie z. B. zu Phenol zu gelangen, ist es nicht möglich, Benzole direkt durch selektive Oxidation mit Luftsauerstoff in einer Stufe und in hohen Ausbeuten in die Phenole zu überführen. Entweder wird der aromatische Ring überhaupt nicht angegriffen oder die Oxidation läuft, da der aromatische Ring durch die Funktionalisierung mit Sauerstoffatomen reaktiver ist als die Ausgangsverbindung Benzol, zum Kohlendioxid durch.

Daher muß die Hydroxylgruppe über Zwischenstufen in das aromatische System eingeführt werden.

Für die Herstellung von Phenolderivaten ausgehend von Benzolderivaten, d. h. aromatischen Kohlenwasserstoffen wird häufig das Cumolverfahren angewandt. Hierbei wird z. B. das aus Benzol und Propen über Alkylierung hergestellte Cumol peroxidiert und danach das Oxidationsprodukt in die beiden Wertprodukte Phenol und Aceton gespalten ("Hock-Verfahren"). Aufgrund seiner Wirtschaftlichkeit hat sich dieses Verfahren weltweit für die Phenolherstellung etabliert.

Sowohl die Herstellung des durch Alkylierung hergestellten Ausgangsprodukts wie z.B. Cumol, Cyclohexylbenzol (Cyclohexylbenzol liefert nach dem "Hoclc-Verfahren" kein Aceton, sondern entsprechend Cyclohexanon) und Cyclododecylbenzol (Cyclododecylbenzol liefert nach dem "Hock-Verfahren" entsprechend Cyclododecanon) als auch die nach dem Oxidationsschritt folgende sauer katalysierte Spaltung und Umlagerung zum Phenol verlaufen in der Regel mit hohen Umsätzen in hoher Selektivität.

Für die Wirtschaftlichkeit des "Hock-Verfahrens" sind die Selektivität der Oxidation des tertiären Kohlenstoffatoms sowie die Reaktionsgeschwindigkeit bzw. der Umsatz daher besonders entscheidend.

Deshalb wurden insbesondere für die Herstellung des Peroxids schon viele Anstrengungen unternommen. In der Praxis hat sich die Oxidation des Ausgangsprodukts mit Luftsauerstoff bewährt. Zusätze wie Radikalstarter oder der Einsatz anderer Oxidationsmittel, z. B. die für die Oxidation von Kohlenwasserstoffen häufig verwendeten Verbindungen KMnO₄, CrO₃ oder HNO₃, beeinträchtigen die Selektivität, führen zu Entsorgungsproblemen, liefern ökologisch bedenkliche Nebenprodukte und korrodieren die Anlage.

Durch den Einsatz von Redoxmetallkatalysatoren ist es möglich, molekularen Sauerstoff für die Oxidation von organischen Verbindungen zu nutzen. Eine ganze Reihe industrieller Verfahren basieren auf der metallkatalisierten Autoxidation von Kohlenwasserstoffen. So erfolgt z. B. die Oxidation von Cyclohexan mit O₂ zu Cyclohexanol bzw. Cyclohexanon unter Verwendung von Kobaltsalzen. Diese Verfahren basieren auf einem Radikalkettenmechanismus. Das Biradikal Sauerstoff reagiert mit einem Kohlenwasserstoffradikal unter Bildung eines Peroxyradikals und anschliessender Kettenfortpflanzung durch Abstraktion eines H-Atoms an einem weiteren Kohlenwasserstoff. Neben Metallsalzen können aber auch organische Moleküle als Radikalstarter fungieren.

Nachteilig bei diesen Verfahren ist, dass die Selektivität mit steigendem Umsatz sehr stark sinkt und deshalb die Verfahren auf niedrigem Umsatzniveau gefahren werden müssen. So wird beispielweise die Oxidation von Cyclohexan zu Cyclohexanol / Cyclohexanon bei einem Umsatz von 10 bis 12 % durchgeführt, damit die Selektivität 80 bis 85 % beträgt ("Industrielle Organische Chemie" 1994, 261, VCH Verlagsgesellschaft mbH, D-69451 Weinheim). In einem weiteren wichtigen industriellen Autoxidationsprozess der Cumol-Oxidation beträgt der Umsatz ca. 30 % bei einer Cumolhydroperoxid-Selektivität von ca. 90 % (Loc. cit. S. 495 ff.).

Eine Alternative zu Metallsalzkatalysatoren stellt die Verwendung von Katalysatorsystemen bzw. Mediatorsystemen wie z. B. N-Hydroxyphthalimid (NHPI) dar. Jedoch ist die Reaktionsgeschwindigkeit bei den in der Literatur beschriebenen Verfahren trotz der hohen Menge an Mediator (bis zu äquimolaren Verhältnissen gegenüber dem Substrat) nicht befriedigend (J. Mol. Catalysis A. 1997, *117*, 123 - 137). So beschreibt US 5 030 739 die Verwendung von N-Hydroxydicarbonsäureimiden zur Oxidation von Isoprenderivaten zu den entsprechenden Acroleinverbindungen. Eine kombinierte Oxidation/Dehydration von Cyclohexadienen bzw. Sechsringsystemen wie α-Terpinen führt zum Cumolderivat, welches jedoch nicht weiter oxidiert wird. Dieses Verfahren ist daher zur Umsetzung von Cumol zu Cumolhydroperoxid nicht geeignet.

Im allgemeinen werden Mengen an Mediator von mindestens 10 Mol-% im Verhältnis zum Substrat eingesetzt, wobei höhere Mengen an Mediator zur Steigerung der Reaktionsgeschwindigkeit verwendet werden (*J. Org. Chem.* 1995, *60,* 3934-3935). Die Produktselektivität ist für eine technische Anwendung nicht ausreichend. So wird bei der Oxidation von Cumol mit NHPI ein Produktgemisch mit Acetophenon als Hauptprodukt erhalten, das gewünschte Oxidationsprodukt Cumolhydroperoxid konnte jedoch nicht isoliert werden (*J. Org. Chem.* **1995**, *60*, 3934-3935).

Eine Weiterentwicklung des Systems stellt die Verwendung von Co-Katalysatoren dar. Als Co-Katalysatoren können Metallverbindungen, insbesondere Schwermetallsalze, Enzyme oder starke Brönsted-Säuren verwendet werden. So zeigten Ishii et al., daß NHPI in Verbindung mit Metallsalzen als Co-Katalysator Vorteile gegenüber der Oxidation mit NHPI ohne Co-Katalysator aufweisen können (z. B. EP 0878234, EP 0864555, EP 0878458, EP 0858835, JP 11180913, J. Mol. Catalysis A. 1997, *117,* 123 - 137). Nachteilig an diesen Systemen ist aber, neben dem unerwünschten Schwermetallgehalt, auch hier die hohe Menge an verwendetem NHPI. Um eine zufriedenstellende Reaktionsgeschwindigkeit zu gewährleisten, müssen mindestens 10 Mol-% an Mediator verwendet werden. Weiterhin nachteilig ist, dass die eingesetzten Redoxmetalle teilweise weitergehende Reaktionen der Produkte katalysieren und so die Selektivität der Reaktion vermindern.

Es sind auch Verfahren bekannt geworden, die nur einen Mediator ohne Co-Katalysator verwenden. Diese sind jedoch auf die Oxidation von besonders aktivierten Substraten wie Ether, Ester oder Isoprenderivate beschränkt.

So wird bei der Oxidation von Cumol mit dem System NHPI/Kobaltacetat ein Produktgemisch aus Acetophenon (Selektivität 54 %), 2-Phenyl-2-propanol (10 %) und Phenol (17 %) erhalten (J. Mol. Catal. A 1997, 117, 123-137). Das gewünschte Produkt Cumolhydroperoxid wird nur intermediär gebildet und ist unter den gegebenen Verfahrensbedingungen nicht stabil. Das angestrebte Wertprodukt Phenol wird im Gegensatz zum primären Oxidationsprodukt Acetophenon in untergeordnetem Maßstab erhalten. EP 0927717 offenbart ein Verfahren zur Herstellung von Cumolhydroperoxid.

Eine weitere Verfahrensvariante stellt die Verwendung von NHPI in Verbindung mit Alkoholen oder Aldehyden dar (Chem. Commun. 1999, 727 - 728, Tetrahedron Letters 1999, 40, 2165 - 2168, Chem Commun. 1997, 447 - 448). Nachteilig an diesen Verfahren ist die Bildung von Koppelprodukten und das verwendete hohe Mediator-Substrat-Verhältnis (10 Mol-%).

In DE 19723890 wird ein Oxidationssystem, bestehend aus einem organischen Mediator und dem Redoxenzym Laccase, für die Herstellung aromatischer und heteroaromatischer Aldehyde und Ketone beschrieben. Auch hier ist die eingesetzte Menge an Mediator sehr hoch. Zudem weist dieses Verfahren durch die Verwendung eines Enzyms ein kompliziertes Reaktionssystem mit einem biologisch notwendigen Puffersystem auf, das die breite Anwendbarkeit dieses Systems einschränkt.
Die Aufgabe der vorliegenden Erfindung bestand darin, ein schwermetallfreies bzw. metallfreies Verfahren zur Herstellung von aromatischen Alkoholen, insbesondere Phenolen durch katalytische Oxidation von Kohlenwasserstoffen zu den Hydroperoxiden mit anschließender Spaltung der Hydroperoxide zu entwickeln, das hohe Selektivitäten bei hohen Umsätzen aufweist.

Überraschenderweise wurde gefunden, daß Verbindungen des Typs 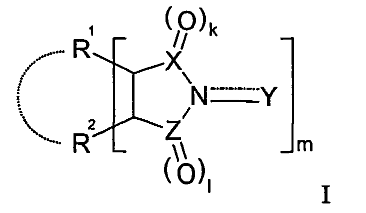 auch ohne Schwermetalle oder starke Säuren als Co-Katalysatoren zur Oxidation von aromatischen Kohlenwasserstoffen zu den entsprechenden Hydroperoxiden eingesetzt werden können.

Gegenstand der vorliegenden Erfindung ist daher ein Verfahren zur Herstellung von Phenolderivaten durch katalytische Oxidation eines aromatischen Kohlenwasserstoffs zum Hydroperoxid und anschließender Spaltung des Hydroperoxids zum Phenolderivat und einem Keton, wobei als Oxidationskatalysator eine Verbindung der Formel I 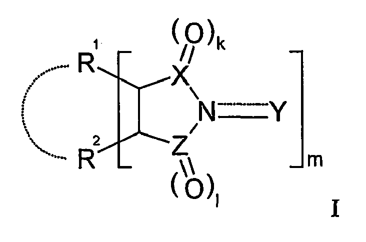
- mit: R¹, R² = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹ und R² identische oder unterschiedliche Reste bezeichnen oder R¹ und R² über eine kovalente Bindung miteinander verknüpft sein können, mit
X, Z = C, S, CH₂
Y = O, OH
k = 0, 1, 2
l = 0, 1, 2
m = 1 bis 3;
unter Anwesenheit eines Radikalstarters, wobei das molare Verhältnis des Oxidationskatalysators zum aromatischen Kohlenwasserstoff unter 10 Mol-% beträgt.

Beispiele für Oxidationskatalysatoren gemäß Formel I sind N-Hydroxyphthalimid, 4-Amino-N-Hydroxyphthalimid, 3-Amino-N-Hydroxyphthalimid, Tetrabromo-N-Hydroxyphthalimid, Tetrachloro-N-Hydroxyphthalimid, N-Hydroxyhetimid, N-Hydroxyhimimid, N-Hydroxytrimellitimid, N-Hydroxy-benzol-1,2,4-tricarbonsäureimid, N,N'-Dihydroxy-pyromellitsäurediimid, N,N'-Dihydroxy-benzophenon-3,3',4,4'tetracarbonsäuredümid, N-Hydroxymaleimid, Pyridin-2,3-dicarbonsäureimid, N-Hydroxysuccinimid, N-Hydroxyweinsäureimid" N-Hydroxy-5-norbonen-2,3-dicarbonsäureimid, exo-N-Hydroxy-7-oxabicyclo[2.2.1]-hept-5-en-2,3-dicarboximid, N-Hydroxy-cis-cyclohexan-1,2-dicarboximid, N-Hydroxy-cis-4-cyclohexen-1,2-dicarbonsäureimid, N-Hydroxynaphthalsäureimid-Natrium-Salz oder N-Hydroxy-o-benzoldisulfonimide.

Im erfindungsgemäßen Verfahren werden keine Metallverbindungen oder Enzyme als Co-Katalysator verwendet. Das Verfahren wird bevorzugt in organischen Lösungsmitteln unter Abwesenheit von starken Säuren durchgeführt, die Verwendung einer wäßrigen Lösung, deren pH-Wert sich im schwach sauren bis basischen Bereich bewegen kann, ist ebenfalls möglich.

Das molare Verhältnis des Oxidationskatalysators zu den aromatischen Kohlenwasserstoffen kann zwischen 10⁻⁶ Mol-% und 10 Mol-%, bevorzugt zwischen 10⁻⁶ und 5 Mol-%, ganz besonders bevorzugt zwischen 10⁻⁶ und 2.5 Mol-% und in einer speziellen Ausführungsform zwischen 10⁻⁶ und 1 Mol-% liegen.

Die Verwendung des Oxidationskatalysators (Mediators) gemäß Formel I in diesem geringen-Verhältnis zu dem zu oxidierenden aromatischen Kohlenwasserstoff hat überraschenderweise nicht nur das Erreichen hohe Umsätze bei kurzen Reaktionszeiten zur Folge, sondern zeichnet sich auch durch die Selektivität gegenüber dem Stand der Technik aus. Ein weiterer Vorteil des erfindungsgemässen Verfahrens besteht in der Verbesserung der Wirtschaftlichkeit durch die Reduktion der Mediatormenge.

Das Verfahren der Erfindung kann ähnlich dem Hock-Prozeß zur Herstellung von Phenol, so beschrieben in "Industrielle Organische Chemie" 1994, 383 ff, VCH Weinheim, durchgeführt werden und kann sich in die Einzelschritte
- Oxidation des aromatischen Kohlenwasserstoffs zum Hydroperoxid
- Isolierung des Hydroperoxids
- Spaltung des Hydroperoxids zum gewünschten Phenolderivat und einem Keton
- Getrennte Isolierung des Phenolderivats und des Ketons gemäß dem folgenden Schema gliedern:

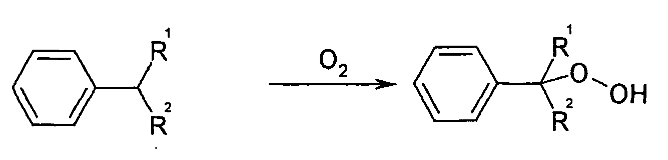

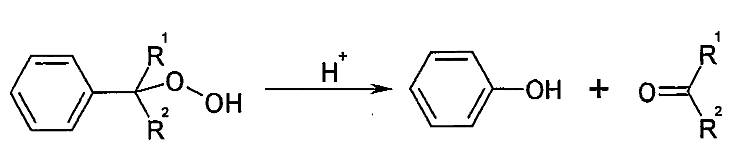

R¹ und R² können z. B. die für die allgemeine Formel IV definierten Bedeutungen haben. Großtechnisch wird im Hock-Verfahren Phenol aus Cumol hergestellt, d. h. R¹ = R² = CH₃.

Die Spaltung des Hydroperoxids kann mit Hilfe einer katalytischen Menge einer Mineralsäure, wie z. B. H₂SO₄ oder einer festen Säure, wie z. B. einem Zeolith, erfolgen.

In speziellen Ausführungsformen des erfindungsgemäßen Verfahrens können auch Derivate bzw. Spezialfälle von Verbindungen der Formel I eingesetzt werden. Diese werden im folgenden mit den Formeln II und II bezeichnet.

Bevorzugt sind Mediatoren oder Oxidationskatalysatoren der Formel II, d. h. Verbindungen gemäß Formel I mit m = 1. Die Bedeutungen von R¹, R², X, Y, Z, k, l gelten entsprechend Formel I

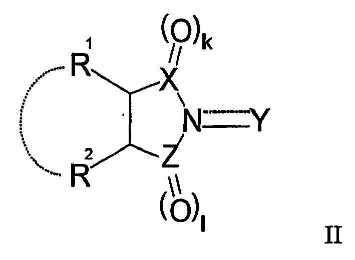

Besonders bevorzugt sind Mediatoren oder Oxidationskatalysatoren der Formel III 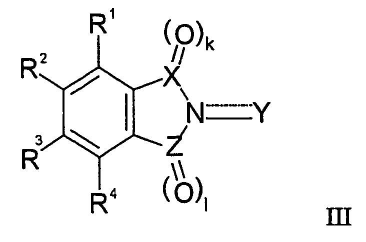
- mit: R¹, R², R³, R⁴ = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹, R², R³ und R⁴ identische oder unterschiedliche Reste bezeichnen können, mit
X, Z = C, S, CH₂
Y = O, OH
k = 0, 1, 2
l = 0, 1, 2.

In besonderen Ausführungsformen der Erfindung erfolgt die Oxidation des aromatischen Kohlenwasserstoffs zu Hydroperoxid in der Gasphase oder in der Flüssigphase jeweils bei einer Temperatur von 0 bis 500 °C, bevorzugt bei einer Temperatur von 50 bis 300 °C und besonders bevorzugt 50 bis 200 °C. Dabei kann sowohl ein Lösemittel bzw. Lösemittelgemisch als auch der aromatische Kohlenwasserstoff selber als Lösemittel verwendet werden.

Die zu oxidierenden Substrate gehören zu der Gruppe der aromatischen Kohlenwasserstoffe. Diese können substituiert oder unsubstituiert sein. Mit Hilfe des erfindungsgemässen Verfahrens können eine Vielzahl dieser Verbindungen selektiv zum Hydroperoxid oxidiert und anschließend zum Phenolderivat weiter umgesetzt werden.

Grundsätzlich können mit dem erfindungsgemäßen Verfahren alle aromatischen Kohlenwasserstoffe mit einem primären, sekundären oder tertiären Kohlenstoffatom zum entsprechenden Hydroperoxid oxidiert werden, bevorzugt werden aber aromatische Kohlenwasserstoffe mit einem sekundären oder tertiären Kohlenstoffatom, besonders bevorzugt werden Verbindungen mit einem tertiären Kohlenstoffatom der Formel IV 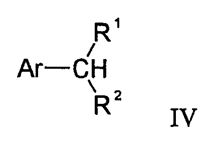
- mit: R¹, R² = aliphatischer oder aromatischer Kohlenwasserstoffrest mit 1 bis 20 Kohlenstoffatomen, wobei R¹ und R² identische oder unterschiedliche Reste bezeichnen und R¹ und R² über eine kovalente Bindung miteinander verknüpft sein können und
- Ar: = aromatischer Kohlenwasserstoffrest
eingesetzt.

Beispiele für Verbindungen gemäß Formel IV sind Cyclohexylbenzol, Cyclododecylbenzol, Ethyl und 2-n-benzol.

Weitere, bevorzugte Substrate für das Verfahren der Erfindung sind Verbindungen der Formeln V, VI, VII, VIII und IX, wobei Ar einen aromatischen Kohlenwasserstoffrest wie z. B. einen Phenylrest (C₆H₅-) bezeichnet

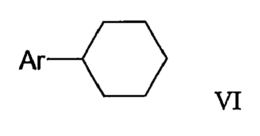

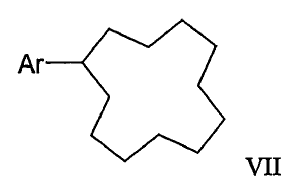

Das Reaktionsgemisch enthält einen Radikalstarter, der unter Bildung von Radikalen, d. h. den radikal-startenden Molekülen zerfällt, wie eine Peroxyverbindung oder eine Azoverbindung.

Beispiele für solche Verbindungen sind Cumolhydroperoxid, Cyclohexylbenzolhydroperoxid, Cyclododecylbenzolhydroperoxid, 1,4-Di(2-neodecanoyl-peroxyisopropyl)benzol, Acetylcyclohexansulfonylperoxid, Cumylperoxyneodecanoat, Dicyclohexylperoxydicarbonat, Di(4-tert.-butylcyclohexyl)peroxydicarbonat, Di(2-ethylhexyl)peroxydicarbonat, Dimyristylperoxydicarbonat, Dicetylperoxydicarbonat, tert.-Butylperoxyneodecanoat, tert.-Amylperoxyneodecanoat, tert.-Amylperoxypivalat, tert.Butylperoxypivalat, Diisononanoylperoxid, Didecanoylperoxid, tert.-Butylperoxy-2-ethylhexanoat, tert.-Butylperoxyisononanoat, 2,2'-Di-tert.Butylperoxybutan, Di-tert.-Butylperoxybenzoat, Di-tert.-Butylperoxid, tert.-Butylhydroperoxid, 3,4-Dimethyl-3,4-diphenylhexan, Dibenzoylperoxid, 1,4-Di-tert.-butylperoxycyclohexan, tert.-Butylperoxyethylhexylcarbonat, 1,1-Di-tert.-butylperoxycyclohexan, 2,2'-Azobis(2,4-dimethylvaleronitril, 2,2'-Azobis(2-methylpropionitril), 1,1'-Azobis(cyclohexancarbonitril) oder Cyclohexylhydroperoxid. Selbstverständlich können auch intermediär gebildete Peroxide und Azoverbindungen als Radikalstarter verwendet werden.

Bevorzugt wird ein Radikalstarter, der ein an ein primäres, sekundäres oder tertiäres Kohlenstoffatom gebundenes Sauerstoffatom enthält, besonders bevorzugt wird ein Radikalstarter, der sich vom Endprodukt ableitet und mindestens ein an ein primäres, sekundäres oder tertiäres Kohlenstoffatom gebundenes Sauerstoffatom enthält. Der Radikalstarter wird entweder separat zugegeben oder wie oben erwähnt während der Reaktion intermediär erzeugt oder er ist sogar, da die Anlage nicht absolut gereinigt werden kann, in geringen Mengen aus vorausgegangenen Reaktionen noch vorhanden. Beispiele für solche Verbindungen sind Cumolhydroperoxid (1-Methyl-1-phenylethylhydroperoxid), Cyclohexylbenzolhydroperoxid (1-Phenylcyclohexylhydroperoxid), Cyclododecylbenzolhydroperoxid (1-Phenylcyclo-dodecylhydroperoxid) und 2-n-Butylbenzolhydroperoxid (1-Methyl-1-phenylpropyl-hydroperoxid).

Die Konzentration des radikal-startenden Moleküls (z. B. Hydroxyradikal) ist im erfindungsgemässen Verfahren zu Beginn der Reaktion häufig geringer als die Konzentration des Katalysators. Es ist jedoch zu beachten, dass im Verlauf der Reaktion eine intermediäre Bildung dieser Verbindungen erfolgt, so dass sich die Konzentration der radikal-startenden Moleküle im Verlauf der Reaktion erhöht.

Das gebildete Oxidationsprodukt kann als solches isoliert werden, aber auch die direkte weitere Umsetzung dieser Verbindung zu einem weiteren Produkt ist möglich. Die Isolation des Produktes ist durch jedes gängige technische Verfahren wie z. B. Destillation möglich.

Das erfindungsgemäße Verfahren kann sowohl batchweise, im fedbatch als auch kontinuierlich durchgeführt werden.

Das gebildete Oxidationsprodukt kann als solches isoliert werden, aber auch die direkte weitere Umsetzung dieser Verbindung zu dem gewünschten Phenolderivat ist möglich.

Das erfindungsgemäße Verfahren kann sowohl batchweise, im fedbatch als auch kontinuierlich durchgeführt werden.

Das erfindungsgemäße Verfahren kann unter Verwendung eines sauerstoffhaltigen Gases als Oxidationsmittel durchgeführt werden. Der Anteil des Sauerstoffs in dem Gas kann zwischen 5 bis 100 Vol-% betragen. Bevorzugt wird Luftsauerstoff oder reiner Sauerstoff als Oxidationsmittel verwendet. Es ist in jedem Fall auf eine innige Vermischung der flüssigen und der gasförmigen Phase zu achten. Dies kann z. B. in Rührkesseln durch eine entsprechende Rührgeschwindigkeit oder durch Einbauten und in Rohrreaktoren mit Packungselementen sowie mit Blasensäuren erreicht werden.

Es ist aber auch möglich, in einer Art Reaktivkolonne zu produzieren. Mittig wäre dann das Katalysatorsystem so angeordnet, dass eine Vermischung mit in der Sumpfphase gebildetem Hydroperoxid nicht möglich ist. Dies kann beispielsweise mit Membranen geeigneter Porengröße erreicht werden. Über Kopf würde nicht umgesetztes Cumol abgetrennt.

Das erfindungsgemässe Verfahren kann sowohl mit geringem Unterdruck als auch unter Atmosphärendruck (1 bar) als auch unter erhöhtem Druck bis zu 100 bar durchgeführt werden. Bevorzugt wird ein Druck von 1 bar bis 50 bar, besonders bevorzugt wird ein Druck von 1 bar bis 20 bar.

Die folgenden Beispiele sollen die Erfindung näher erläutern, ohne ihren Schutzumfang einzuschränken.

Der Umsatz der Oxidation wurde zum einen durch die Titration des Peroxids mit Iod und zum anderen durch die GC-Analyse mit einem internen Standard (Naphthalin) bestimmt. Die Selektivität der Oxidationsreaktion wurde ebenfalls durch GC-Analyse mit einem internen Standard (ebenfalls Naphthalin) bestimmt. Der Umsatz und die Selektivität der Spaltungsreaktion wurde durch GC-Analyse mit einem internen Standard (Naphthalin) bestimmt. Die Selektivität der Spaltungsprodukte ist immer auf das zu oxidierende Ausgangsprodukt bezogen.

### Beispiel 1a (Vergleich):

30 mmol Cumol werden in einem Rundkolben mit aufgesetztem Rückflusskühler bei einer Temperatur von 125 °C mit 0,3 mmol N-Hydroxyphthalimid und 0,6 mmol Cumolhydroperoxid versetzt. Das Reaktionsgemisch wird 8 Stunden bei der genannten Temperatur unter einer Sauerstoffatmosphäre von 1 bar gerührt. Es wird Cumolhydroperoxid mit einer Selektivität von 99,9 % bei einem Cumol-Umsatz von 30,8 % erhalten.

### Beispiel 1b (Vergleich):

Der Reaktionsaustrag der Oxidation (Beispiel 1a) wird am Rotationsverdampfer durch Verdampfung von nicht umgesetzten Cumol aufkonzentriert. Der Sumpf enthält 70 Gew.-% Cumolhydroperoxid.
Das Konzentrat wird in 10 ml Aceton aufgenommen und bei 50 °C zu einem Gemisch aus 90 ml Aceton und einer geringen Menge Schwefelsäure (1500 ppm) zudosiert. Nach 15 Minuten wird die Zusammensetzung des Reaktionsgemisches mittels GC bestimmt. Bei einem quantitativen Umsatz von Cumolhydroperoxid wird Phenol mit einer Selektivität von 92 % und Aceton mit einer Selektivität von 91 % erhalten

### Beispiel 2 (nicht erfindungsgemäß, mit Co-Katalysator):

30 mmol Cumol werden in einem Rundkolben mit aufgesetztem Rückflusskühler bei einer Temperatur von 125 °C mit 0,3 mmol N-Hydroxyphthalimid und 0,3 mmol Co(II)-Acetat versetzt. Das Reaktionsgemisch wird 8 Stunden bei der genannten Temperatur unter einer Sauerstoffatmosphäre von 1 bar gerührt. Es wird nicht das Zielprodukt, sondern Acetophenon mit einer Selektivität von 58,7 %, 2-Phenyl-2-propanol (13,1 %) und Phenol (10,4 %) bei einem Cumol-Umsatz von 49,3 % erhalten.

### Beispiel 3a (erfindungsgemäss):

30 mmol Cyclohexylbenzol werden in einem Rundkolben mit aufgesetztem Rückflusskühler bei einer Temperatur von 110°C mit 0,3 mmol N-Hydroxyphthalimid und 0,6 mmol 1-Cyclohexybenzylhydroperoxid versetzt. Das Reaktionsgemisch wird 8 Stunden bei der genannten Temperatur unter einer Sauerstoffatmosphäre von 1 bar gerührt. Es wird 1-Cyclohexylbenzolhydroperoxid mit einer Selektivität von 96,2 % bei einem Cyclohexylbenzol-Umsatz von 28,6 % erhalten.

### Beispiel 3b (erfindungsgemäss):

Der Reaktionsaustrag der Oxidation (Beispiel 3a) wird am Rotationsverdampfer durch Verdampfung von nicht umgesetzten Cyclohexylbenzol aufkonzentriert. Der Sumpf enthält 65 Gew.-% 1-Cyclohexylbenzolhydroperoxid.

Das Konzentrat wird in 10 ml Aceton aufgenommen und bei 50°C zu einem Gemisch aus 90 ml Aceton und einer geringen Menge Schwefelsäure (1500 ppm) zudosiert. Nach 30 Minuten wird die Zusammensetzung des Reaktionsgemisches mittels GC bestimmt. Bei einem quantitativen Umsatz von 1-Cyclohexylbenzolhydroperoxid wird Phenol mit einer Selektivität von 88 % und Cyclohexanon mit einer Selektivität von 91 % erhalten

### Beispiel 4a (nicht erfindungsgemäß, mit Co-Katalysator):

30 mmol Cyclohexylbenzol werden in einem Rundkolben mit aufgesetztem Rückflusskühler bei einer Temperatur von 110 °C mit 0,3 mmol N-Hydroxyphthalimid und 0,3 mmol Co(II)-Acetat versetzt. Das Reaktionsgemisch wird 8 Stunden bei der genannten Temperatur unter einer Sauerstoffatmosphäre von 1 bar gerührt. Es wird 1-Cyclohexylbenzolhydroperoxid mit einer Selektivität von 54,1 % bei einem Cyclohexylbenzol-Umsatz von 18,7 % erhalten.

### Beispiel 4b (nicht erfindungsgemäss):

Der Reaktionsaustrag der Oxidation (Beispiel4a) wird am Rotationsverdampfer durch Verdampfung von nicht umgesetzten Cyclohexylbenzol aufkonzentriert. Der Sumpf enthält 35 Gew. -% 1-Cyclohexylbenzolhydroperoxid.
Das Konzentrat wird in 10 ml Aceton aufgenommen und bei 50 °C zu einem Gemisch aus 90 ml Aceton und einer geringen Menge Schwefelsäure (1500 ppm) zudosiert. Nach 30 Minuten wird die Zusammensetzung des Reaktionsgemisches mittels GC bestimmt. Bei einem quantitativen Umsatz von 1-Cyclohexylbenzolhydroperoxid wird Phenol mit einer Selektivität von 30 % und Cyclohexanon mit einer Selektivität von 32 % erhalten

### Beispiel 5a (erfindungsgemäss):

30 mmol Cyclododecylbenzol werden in einem Rundkolben mit aufgesetztem Rückflusskühler bei einer Temperatur von 125 °C mit 0,3 mmol N-Hydroxyphthalimid und 0,6 mmol Cyclododecylbenzolhydroperoxid versetzt. Das Reaktionsgemisch wird 8 Stunden bei der genannten Temperatur unter einer Sauerstoffatmosphäre von 1 bar gerührt. Es wird Cyclododecylbenzolhydroperoxid mit einer Selektivität von 95,1 % bei einem Cyclododecylbenzol-Umsatz von 23,1 % erhalten.

### Beispiel 5b (erfindungsgemäss):

Der Reaktionsaustrag der Oxidation (Beispiel 5a) wird am Rotationsverdampfer durch Verdampfung von nicht umgesetzten Cyclododecylbenzol aufkonzentriert. Der Sumpf enthält 60 Gew.-% Cyclododecylbenzolhydroperoxid.
Das Konzentrat wird in 10 ml Aceton aufgenommen und bei 50 °C zu einem Gemisch aus 90 ml Aceton und einer geringen Menge Schwefelsäure (1500 ppm) zudosiert. Nach 35 Minuten wird die Zusammensetzung des Reaktionsgemisches mittels GC bestimmt. Bei einem quantitativen Umsatz von Cyclododecylbenzolhydroperoxid wird Phenol mit einer Selektivität von 87 % und Cyclododecanon mit einer Selektivität von 90 % erhalten

### Beispiel 6a (nicht erfindungsgemäß, mit Co-Katalysator):

30 mmol Cyclododecylbenzol werden in einem Rundkolben mit aufgesetztem Rückflusskühler bei einer Temperatur von 125 °C mit 0,3 mmol N-Hydroxyphthalimid und 0,3 mmol Co(II)-Acetat versetzt. Das Reaktionsgemisch wird 8 Stunden bei der genannten Temperatur unter einer Sauerstoffatmosphäre von 1 bar gerührt. Es wird Cyclododecylbenzolhydroperoxid mit einer Selektivität von 59,1 % bei einem Cyclododecylbenzol-Umsatz von 7,3 % erhalten.

### Beispiel 6b (nicht erfindungsgemäss):

Der Reaktionsaustrag der Oxidation (Beispiel 6a) wird am Rotationsverdampfer durch Verdampfung von nicht umgesetzten Cyclododecylbenzol aufkonzentriert. Der Sumpf enthält 33 Gew.-% Cyclododecylbenzolhydroperoxid.
Das Konzentrat wird in 10 ml Aceton aufgenommen und bei 50 °C zu einem Gemisch aus 90 ml Aceton und einer geringen Menge Schwefelsäure (1500 ppm) zudosiert. Nach 35 Minuten wird die Zusammensetzung des Reaktionsgemisches mittels GC bestimmt. Bei einem quantitativen Umsatz von Cyclododecylbenzolhydroperoxid wird Phenol mit einer Selektivität von 28 % und Cyclododecanon mit einer Selektivität von 32 % erhalten

## Patentansprüche

1. Verfahren zur Herstellung von Phenolderivaten durch katalytische Oxidation eines aromatischen Kohlenwasserstoffs zum Hydroperoxid und anschließender Spaltung des Hydroperoxids zum Phenolderivat und einem Keton, wobei als aromatischer Kohlenwasserstoff eine Verbindung der Formel IV 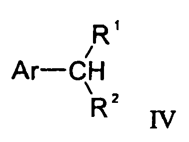
mit R¹, R² = aliphatischer Kohlenwasserstoffrest mit 1 bis 20 Kohlenstoffatomen, wobei R¹ und R² identische oder unterschiedliche Reste bezeichnen und R¹ und R² über eine kovalente Bindung miteinander verknüpft sind und
Ar = aromatischer Kohlenwasserstoffrest
eingesetzt wird,
**dadurch gekennzeichnet,**
**dass** als Oxidationskatalysator eine Verbindung der Formel I 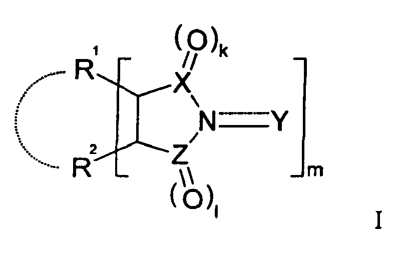
mit R¹, R² = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹ und R² identische oder unterschiedliche Reste bezeichnen oder R¹ und R² über eine kovalente Bindung miteinander verknüpft sein können, mit
X, Z = C, S, CH₂
Y = O, OH
k =0, 1, 2
l =0, 1, 2
m = 1 bis 3;
unter Anwesenheit eines Radikalstarters eingesetzt wird, wobei das molare Verhältnis des Katalysators zum aromatischen Kohlenwasserstoff unter 10 Mol-% beträgt.

2. Verfahren nach Anspruch 1,
**dadurch gekennzeichnet,**
**dass** als Oxidationskatalysator eine Verbindung der Formel III 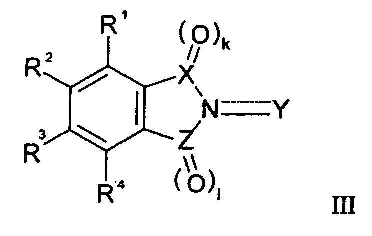
mit R¹, R², R³, R⁴ = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹, R², R³ und R⁴ identische oder unterschiedliche Reste bezeichnen können, mit
X, Z = C, S, CH₂
Y = O, OH
k = 0, 1, 2
l = 0, 1, 2.
eingesetzt wird.

3. Verfahren nach einem der Ansprüche 1 oder 2,
**dadurch gekennzeichnet,**
**dass** das molare Verhältnis des Oxidationskatalysators zu dem zu oxidierenden, aromatischen Kohlenwasserstoff zwischen 10⁻⁶ Mol-% und 10 Mol-% liegt.

4. Verfahren nach Anspruch 3,
**dadurch gekennzeichnet,**
**dass** das molare Verhältnis des Oxidationskatalysators zu dem zu oxidierenden, aromatischen Kohlenwasserstoff zwischen 10⁻⁶ Mol-% und 2,5 Mol-% liegt.

5. Verfahren nach einem der Ansprüche 1 bis 4,
**dadurch gekennzeichnet,**
**dass** als Radikalstarter eine Peroxyverbindung oder Azoverbindung verwendet wird.

6. Verfahren nach Anspruch 5,
**dadurch gekennzeichnet,**
**dass** Radikalstarter und Oxidationskatalysator im Molverhältnis 4 : 1 eingesetzt werden.

7. Verfahren nach einem der Ansprüche 1 bis 6,
**dadurch gekennzeichnet,**
**dass** die katalytische Oxidation in der Flüssigphase bei einer Temperatur von 0 bis 500 °C durchgeführt wird.

8. Verfahren nach einem der Ansprüche 1 bis 7,
**dadurch gekennzeichnet,**
**dass** als Oxidationsmittel ein Gas mit 5 bis 100 Vol.-% Sauerstoff verwendet wird.

9. Verfahren nach einem der Ansprüche 1 bis 8,
**dadurch gekennzeichnet,**
**dass** die katalytische Oxidation unter einem Druck von 1 bis 100 bar durchgeführt wird.

10. Verfahren nach einem der Ansprüche 1 bis 9,
**dadurch gekennzeichnet,**
**dass** als aromatischer Kohlenwasserstoff Verbindungen der Formeln VI und VII 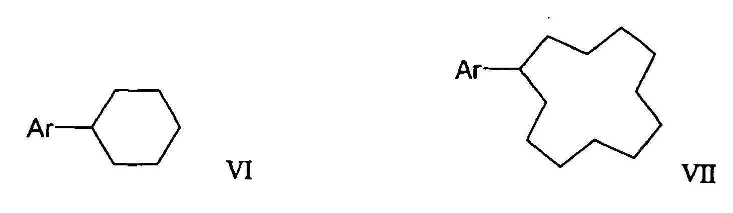 mit Ar = aromatischer Kohlenwasserstoffrest
eingesetzt werden.

## Claims

1. Process for preparing phenol derivatives by catalytic oxidation of an aromatic hydrocarbon to the hydroperoxide and subsequent cleavage of the hydroperoxide to give the phenol derivative and a ketone, where the aromatic hydrocarbon used is a compound of the formula IV 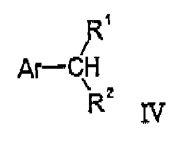 where R¹, R² = aliphatic hydrocarbon radical having from 1 to 20 carbon atoms, where R¹ and R² are identical or different radicals and R¹ and R² are joined to one another via a covalent bond and
Ar = aromatic hydrocarbon radical,
**characterized in that** a compound of the formula I 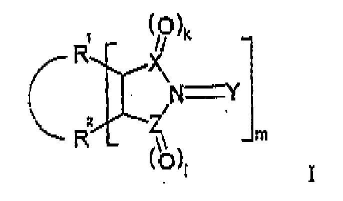 where R¹, R² = H, aliphatic or aromatic alkoxy radical, carboxyl radical, alkoxycarbonyl radical or hydrocarbon radical, in each case having from 1 to 20 carbon atoms, SO₃H, NH₂, OH, F, Cl, Br, I and/or NO₂, where R¹ and R² are identical or different radicals or R¹ and R² may be joined to one another via a covalent bond, and
X, Z = C, S, CH2
Y = O, OH
k = 0, 1, 2
l = 0, 1, 2
m = 1-3;
is used as oxidation catalyst in the presence of a free-radical initiator, where the molar ratio of the catalyst to the aromatic hydrocarbon is less than 10 mol%.

2. Process according to Claim 1, **characterized in that** the oxidation catalyst used is a compound of the formula III 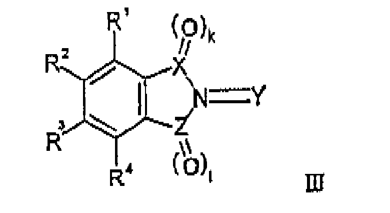 where R¹, R², R³, R⁴ = H, aliphatic or aromatic alkoxy radical, carboxyl radical, alkoxycarbonyl radical or hydrocarbon radical, in each case having from 1 to 20 carbon atoms, SO₃H, NH₂, OH, F, Cl, Br, I and/or NO₂, where R¹, R², R³ and R⁴ may be identical or different radicals, and
X, Z = C, S, CH2
Y = O, OH
k = 0, 1, 2
l = 0, 1, 2.

3. Process according to Claim 1 or 2, **characterized in that** the molar ratio of the oxidation catalyst to the aromatic hydrocarbon to be oxidized is from 10⁻⁶ mol% to 10 mol%.

4. Process according to Claim 3, **characterized in that** the molar ratio of the oxidation catalyst to the aromatic hydrocarbon to be oxidized is from 10⁻⁶ mol% to 2.5 mol%.

5. Process according to any of Claims 1 to 4, **characterized in that** the free-radical initiator used is a peroxy compound or azo compound.

6. Process according to Claim 5, **characterized in that** free-radical initiator and oxidation catalyst are used in a molar ratio of 4:1.

7. Process according to any of Claims 1 to 6, **characterized in that** the catalytic oxidation is carried out in the liquid phase at a temperature of from 0 to 500°C.

8. Process according to any of Claims 1 to 7, **characterized in that** the oxidant used is a gas containing from 5 to 100% by volume of oxygen.

9. Process according to any of Claims 1 to 8, **characterized in that** the catalytic oxidation is carried out under a pressure of from 1 to 100 bar.

10. Process according to any of Claims 1 to 9, **characterized in that** the aromatic hydrocarbon used is a compound of the formula VI or VII 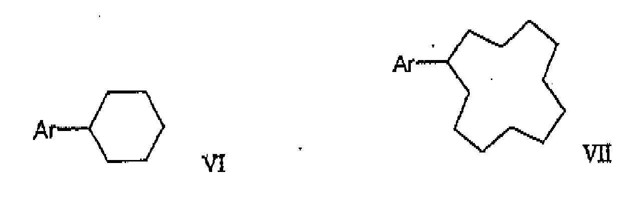 where Ar = aromatic hydrocarbon radical.

## Revendications

1. Procédé de préparation de dérivés de phénol par oxydation catalytique d'un hydrocarbure aromatique en hydroperoxyde et dissociation consécutive de l'hydroperoxyde en dérivé du phénol et une cétone, en utilisant comme hydrocarbure aromatique un composé de formule IV 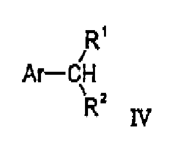 avec R¹, R² = un radical hydrocarboné aliphatique comprenant 1 à 20 atomes de carbone, R¹ et R² désignant des radicaux identiques ou différents et R¹ et R² étant reliés l'un avec l'autre via une liaison covalente et Ar = un radical hydrocarboné aromatique
**caractérisé en ce qu'**on utilise comme catalyseur d'oxydation un composé de formule I 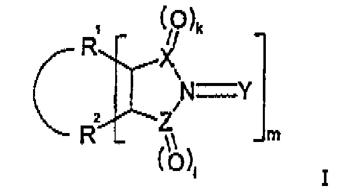 avec R¹, R² = H, un radical alcoxy aliphatique ou aromatique, un radical carboxyle, un radical alcoxycarbonyle ou un radical hydrocarboné, comprenant à chaque fois 1 à 20 atomes de carbone, SO₃H, NH₂, OH, F, Cl, Br, I et/ou NO₂, R¹ et R² désignant des radicaux identiques ou différents ou R¹ et R² pouvant être reliés l'un à l'autre via une liaison covalente, avec
X, Z = C, S, CH₂
Y = O, OH
k = 0, 1, 2
l = 0, 1, 2
m = 1 à 3 ;
en présence d'un initiateur radicalaire, le rapport molaire du catalyseur au radical hydrocarboné aromatique étant inférieur à 10% en mole.

2. Procédé selon la revendication 1, **caractérisé en ce qu'**on utilise comme catalyseur d'oxydation un composé de formule III 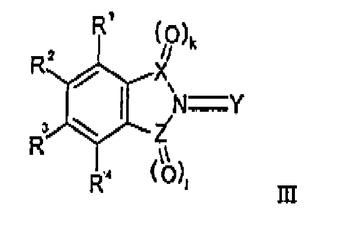 avec R¹, R², R³, R⁴ = H, un radical alcoxy aliphatique ou aromatique, un radical carboxyle, un radical alcoxycarbonyle ou un radical hydrocarboné, comprenant à chaque fois 1 à 20 atomes de carbone, SO₃H, NH₂, OH, F, Cl, Br, I et/ou NO₂, R¹, R², R³ et R⁴ pouvant désigner des radicaux identiques ou différents, avec
X, Z = C, S, CH₂
Y = O, OH
k = 0, 1, 2
l = 0, 1, 2.

3. Procédé selon l'une quelconque des revendications 1 ou 2, **caractérisé en ce que** le rapport molaire du catalyseur d'oxydation à l'hydrocarbure aromatique à oxyder est compris entre 10⁻⁶% en mole et 10% en mole.

4. Procédé selon la revendication 3, **caractérisé en ce que** le rapport molaire du catalyseur d'oxydation à l'hydrocarbure aromatique à oxyder est compris entre 10⁻⁶% en mole et 2,5% en mole.

5. Procédé selon l'une quelconque des revendications 1 à 4, **caractérisé en ce qu'**on utilise comme initiateur radicalaire un composé peroxy ou un composé azo.

6. Procédé selon la revendication 5, **caractérisé en ce qu'**on utilise l'initiateur radicalaire et le catalyseur d'oxydation dans un rapport molaire de 4 : 1.

7. Procédé selon l'une quelconque des revendications 1 à 6, **caractérisé en ce que** l'oxydation catalytique est réalisée en phase liquide à une température de 0 à 500°C.

8. Procédé selon l'une quelconque des revendications 1 à 7, **caractérisé en ce qu'**on utilise comme oxydant un gaz contenant 5 à 100% en volume d'oxygène.

9. Procédé selon l'une quelconque des revendications 1 à 8, **caractérisé en ce que** l'oxydation catalytique est réalisée sous une pression de 1 à 100 bars.

10. Procédé selon l'une quelconque des revendications 1 à 9, **caractérisé en ce qu'**on utilise comme hydrocarbure aromatique les composés des formules VI et VII 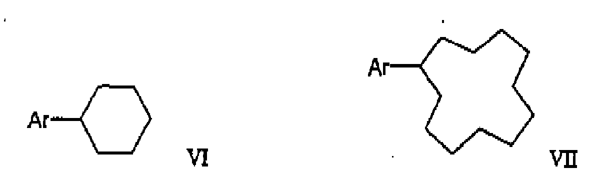 avec Ar = un radical hydrocarboné aromatique.
